# EUROPEAN PATENT SPECIFICATION

(11) **EP 3 727 359 B1**
(45) Date of publication and mention of the grant of the patent: **17.04.2024**
(21) Application number: 18822360.6
(22) Date of filing: 20.12.2018
(51) Int. Cl.: A61K 31/047, A61P 43/00

(54) **TREATMENT OF FIBROSIS WITH INOSITOL**
BEHANDLUNG VON FIBROSE MIT INOSITOL
TRAITEMENT DE LA FIBROSE AVEC DE L'INOSITOL

(30) Priority: 22.12.2017 IT 201700148902
(43) Date of publication of application: 28.10.2020
(73) Proprietor: LO.LI. Pharma S.r.l., 00156 Roma (RM) (IT)
(72) Inventor: UNFER, Vittorio, 00155 Rome (IT)
(74) Representative: Capasso, Olga
(86) International application number: PCT/EP2018/086182
(87) International publication number: WO 2019/122119

(56) References cited:
- WO-A1-2011/005886
- WO-A1-2017/187375
- US-B1- 6 245 755
- V. PASTA ET AL: "An association of boswellia, betaine and myo-inositol (Eumastos) in the treatment of mammographic breast density: a randomised, double-blind study", EUROPEAN REVIEW FOR MEDICAL AND PHARMACOLOGICAL SCIENCES, vol. 19, 2015, pages 4419-4426, XP002784477,

## Description

### Field of the invention

The present invention relates to inositol for use in the treatment of reactive muscle fibrosis, e.g. reactive cardiac fibrosis, in a subject. The present invention further relates to compositions for use in methods of treating reactive muscle fibrosis, e.g. reactive cardiac fibrosis by administering inositol to a subject.

### Background of the invention

Fibrosis, in general, is a scarring process which is characterized by fibroblast accumulation and excess deposition of extracellular matrix (ECM) proteins, leading to distorted organ architecture and function. The formation of excess fibrous connective tissue in an organ or tissue can occur in a reactive or reparative process. Reactive fibrosis is a reversible process that appears in the absence of functional cell necrosis (i.e. the affected cells remain alive), whereas reparative fibrosis is accompanied by scar formation after cell death.

Metabolic dysregulation is generally linked to a chronic low-grade inflammation state that has been called "meta-inflammation" (short for "metabolic inflammation"). One means by which meta-inflammation is linked to metabolic disorders is extracellular matrix expansion (Daniel S. Lark and David H. Wasserman; F1000Research 27(6), 1758, 2017). In metabolic disorders, various organs and tissues can be affected by reactive fibrosis, in which functional cells are not substituted by fibroblasts but excess deposition of extracellular matrix (ECM) proteins and increased amounts of fibroblasts occur, leading to altered organ and tissue function.

Among different metabolic disorders, metabolic syndrome (MetS) and obesity are growing causes of morbidity and mortality worldwide. MetS is a major risk factor for cardiovascular disease and is defined by the National Institutes of Health by having at least 3 of the following conditions: hypertension, elevated fasting plasma glucose, central obesity, elevated triglycerides, or low high-density lipoprotein cholesterol. Each component of MetS causes cardiac dysfunction and their combination carries additional risk. The mechanisms underlying cardiac dysfunction in the MetS are complex and include lipid accumulation, increased fibrosis and stiffness, altered calcium homeostasis, mitochondrial dysfunction and increased oxidative stress.

Overweight and obesity are defined as abnormal or excessive fat accumulation that may impair health. Body mass index (BMI) is a simple index of weight-for-height that is commonly used to classify overweight and obesity in adults. It is defined as a person's weight in kilograms divided by the square of his height in meters (kg/m²).

For adults, WHO defines overweight and obesity as follows: overweight corresponds to a BMI from 25 kg/m² up to but not including 30 kg/m², while obesity corresponds to a BMI greater than or equal to 30 kg/m². Obesity is associated with metabolic dysfunctions; fasting hyperglycemia, insulin resistance and dyslipidemias are highly prevalent in obese subjects and stimulate a chronic low-grade inflammatory state that is critically involved in fibrotic remodelling.

A body of evidence links both MetS and obesity to the development of cardiac fibrosis, which is a significant global health problem and may contribute to the increased incidence of heart failure, atrial arrhythmias and sudden cardiac death in subjects. The development of myocardial fibrosis (i.e. cardiac fibrosis) is related to a hypertrophic process and is characterized by an accumulation of collagen type I and III within the interstitial space and the adventitia of intramyocardial coronary arteries. This process, appearing only in the absence of myocyte necrosis, is termed interstitial and perivascular fibrosis (each of which is a type of "reactive fibrosis"). Later fibrosis is accompanied by scar formation after myocyte death (known as "reparative fibrosis"). During the process of reactive fibrosis, fibrillar collagen accumulates initially around coronary arteries (perivascular fibrosis) and later begins to radiate outward into neighboring intermuscular spaces (interstitial fibrosis). The fibrotic ECM causes increased stiffness and induces pathological signaling within cardiomyocytes resulting in progressive cardiac failure. Also, the excessive ECM impairs mechano-electric coupling of cardiomyocytes and increases the risk of arrhythmias.

Several molecular processes have been implicated in the regulation of the meta-fibrotic response, that is a fibrotic development in response to metabolic aberrations or pathologies: Activation of the Renin-Angiotensin-Aldosterone System, induction of Transforming Growth Factor-β, oxidative stress, advanced glycation end-products (AGEs), endothelin-1, Rho-kinase signaling, leptin-mediated actions and upregulation of matricellular proteins (such as thrombospondin-1) play a key role in the development of fibrosis in models of obesity and metabolic dysfunction.

Cardiac fibrosis may play an important role in the pathogenesis of heart disease in patients through several distinct mechanisms. First, fibrotic myocardial remodeling may impair ventricular diastolic function contributing the development of heart failure with preserved ejection fraction. Second, development of atrial fibrosis may predispose subjects to atrial tachyarrhythmias. Third, collagen deposition in the ventricular myocardium may contribute to the increased incidence of ventricular arrhythmias and sudden death observed in dysmetabolic individuals. Fourth, in some patients, development of fibrotic changes in the right ventricle may perturb right ventricular function. Finally, dysmetabolic-related modulation of the reparative fibrotic response following infarction may predispose these subjects to development of post-infarction heart failure. Thus, attenuation of fibrosis is likely to have profound beneficial effects in reducing cardiac morbidity and mortality in subjects with altered metabolism.

Weight loss, achieved either via bariatric surgery or through diet, reverses many structural and functional cardiac abnormalities. However, in several studies, despite significant weight loss, the functional consequences of dysmetabolism persist. Strategies aimed at prevention and treatment of reactive muscle fibrosis, in particular cardiac fibrosis, may be particularly valuable in these patients.

Inositols are a family of simple carbohydrates naturally found in several foods. Inositols exist in 9 possible stereoisomers, 2 of which are predominantly found in eukaryotic cells: myoinositol and D-chiroinositol. It is well established that myoinositol and D-chiroinositol have different roles which lead to different functions within the cells. Myoinositol is the precursor of inositol triphosphate, a second messenger which is responsible for the regulation of many hormones such as insulin, thyroid-stimulating hormone, and follicle-stimulating hormone. D-chiroinositol is the conversion product of myoinositol, by an epimerization reaction which is unidirectional and insulin-dependent. D-chiroinositol is able to regulate the intracellular accumulation of glucose (ie, glycogen synthesis).

The physiological plasma ratio of myoinositol and D-chiroinositol has been reported to be equal to 40:1 and this mixture corresponds to the ratio which has been proven to be the most effective on several clinical trials of conditions such as polycystic ovary syndrome (PCOS). So far, clinical evidence of the use of mixtures of myoinositol and D-chiroinositol has related to the treatment of PCOS and gestational diabetes. To the Applicant's knowledge, no evidence has ever been reported relating to the possible beneficial effects of inositol or inositols on fibrotic tissue reversion in general or treatment of cardiac fibrosis in particular.

Inositol is known for use in the treatment of breast tissue fibrosis (see V. PASTA ET AL: "An association of boswellia, betaine and myo-inositol (Eumastos) in the treatment of mammographic breast density: a randomised, double-blind study", EUROPEAN REVIEW FOR MEDICAL AND PHARMACOLOGICAL SCIENCES, vol. 19, 2015, pages 4419-4426).

WO 2011/005886 A1 discloses the use of inositol in the treatment of lung fibrosis.

US 6 245 755 B1 discloses the treatment of cardiac fibrosis with aldosterone antagonists.

An object of this invention is to advantageously extend the range of the use of inositol and inositol mixtures to the treatment of conditions beyond e.g. PCOS. The present invention addresses this goal.

### Summary of the invention

Accordingly, one aspect of the invention provides inositol for use in a method of treating or preventing reactive muscle fibrosis, for example cardiac fibrosis, in a subject. A further aspect of the invention relates to a composition for use in a method of treating or preventing reactive muscle fibrosis, for example cardiac fibrosis, in a subject in need or potential need thereof, said method comprising administering inositol to said subject.

The Applicant has surprisingly found that in treating MetS or obesity mice models with inositol or a mixture of different inositol isomers, advantageous effects in the treatment of reactive muscle fibrosis, especially cardiac fibrosis, in particular perivascular cardiac fibrosis and interstitial cardiac fibrosis were achieved. Up to now, it is been known that administration of inositol, or a mixture of inositol isomers, could be used to treat diseases such as PCOS, but it was not known or suspected that such compound(s) could be advantageous in the treatment of other diseases, in particular reactive muscle fibrosis, especially cardiac fibrosis, and the Applicant is aware of no experimental findings of this nature. Evidence of the reduction of reactive muscle fibrosis, in particular cardiac fibrosis, in particular perivascular cardiac fibrosis, interstitial cardiac fibrosis, or both perivascular and interstitial cardiac fibrosis, represents an absolutely novel effect advantageously achievable using inositol, for instance myoinositol, or a mixture of inositol isomers, for example myoinositol and at least one other inositol isomer.

### Description of the figures

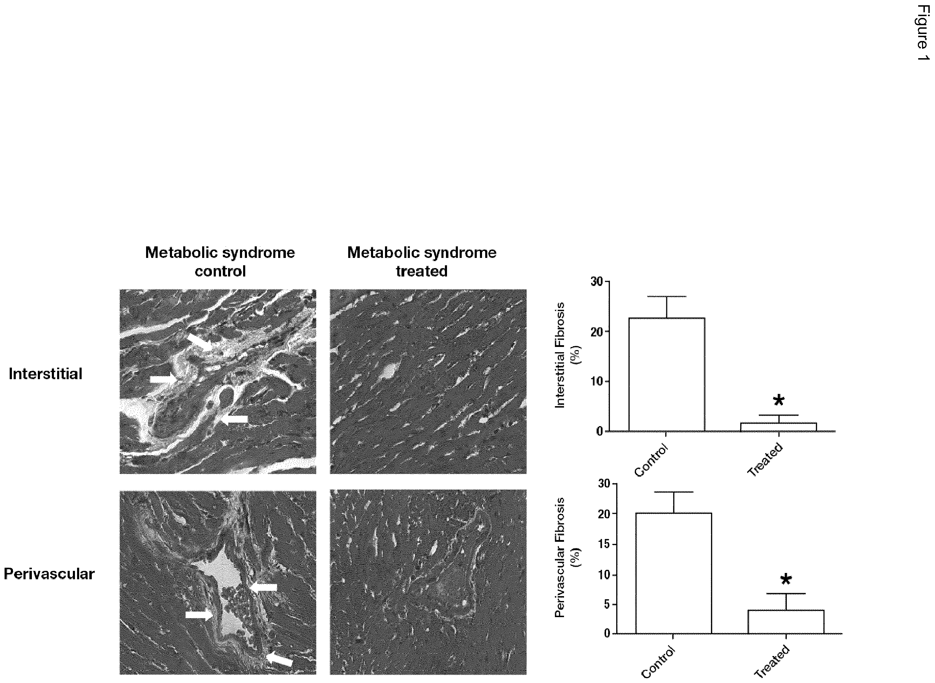

Figure 1 shows, on the left, 4 photographs from a histological analysis of cardiac tissue from representative pregnant mice used as models for MetS (magnification 40x). On the right, Figure 1 shows a bar chart quantifying the observations gained from the histological analysis shown, for 5 animals (mean ± standard error measurement). Specifically, the histological analysis on the left compares, for interstitial (top two photographs) and perivascular (bottom two photographs) fibrosis in pregnant mice modeling MetS, the degree of cardiac fibrosis in mice receiving a placebo control (left photographs) or treatment with myoinositol (MI) and D- -chiroinositol (DCI) in a respective weight ratio of 40:1 (right photographs). Healthy cardiac tissue is shown in darker color, while diseased fibrotic tissue shows in lighter color (illustrated by overlaid white arrows). As is clear in comparing the panels showing cardiac tissue from untreated (left) and treated (right) mice, both intestitial (top) and perivascular (bottom) fibrosis are significantly more pronounced in untreated mice than in mice treated with a mixture of MI and DCI in a respective approximate weight ratio of 40:1. The bar charts on the right of Figure 1 quantify these findings for the histological data shown for perivascular fibrosis (bottom) and interstitial fibrosis (top) . With regard to perivascular fibrosis (bottom bar chart in Figure 1 and Table 1 in Example 1), fibrotic cardiac tissue accounted for 19.9% of all tissue analyzed in the untreated group. In contrast, the group treated with a combination of MI and DCI in a respective approximate weight ratio of 40:1 accounted for only 4.67% of all tissue analyzed, corresponding to a decrease of perivascular cardiac fibrosis by about 77% in the treatment group relative to the untreated control. Similarly quantified are the results of a histological study showing the decrease in the amount of interstitial cardiac fibrosis between untreated and treated mouse groups, wherein the treated mouse group was treated identically to that described above (top bar chart in Figure 1 and Table 1 in Example 1). As can be seen from the top bar chart in Figure 1, interstitial cardiac fibrosis decreased from 21.1% of total cardiac tissue analyzed in the untreated group to 2.57% of total cardiac tissue analyzed in the group treated with MI and DCI, corresponding to a decrease of interstitial cardiac fibrosis by about 88% relative to the untreated control group.

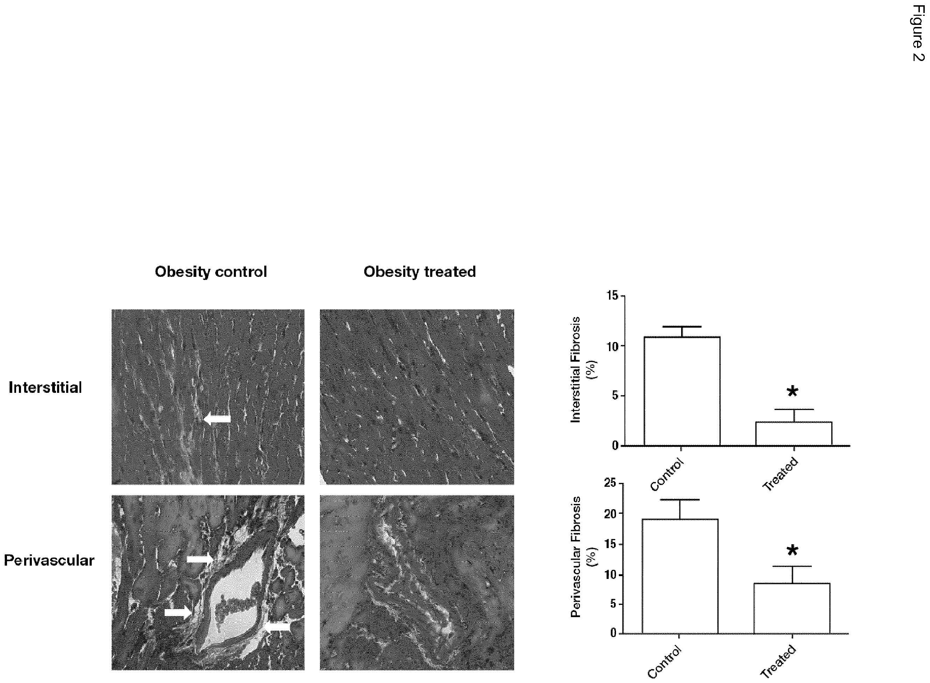

Figure 2 shows, on the left, 4 photographs from histological analyses of cardiac tissue from representative mice used as models for obesity (magnification 40x). On the right, Figure 2 shows a bar chart quantifying the observations gained from the histological analyses shown for 6 animals (mean ± standard error measurement). Specifically, the histological analysis on the left compares, for interstitial (top two photographs) and perivascular (bottom two photographs) fibrosis in pregnant mice modeling obesity, the degree of cardiac fibrosis in mice receiving a placebo control (left photographs) or treatment with M! and DCI in a respective weight ratio of 40:1 (right photographs). Healthy cardiac tissue is shown in darker color, while diseased fibrotic tissue is shown in lighter color (illustrated by white overlaid arrows). As is clear in comparing the panels showing cardiac tissue from control (left) and treated (right) mice, both interstitial (top) and perivascular (bottom) fibrosis is significantly more pronounced in untreated mice than in mice treated with a mixture of MI and DCI in a respective approximate weight ratio of 40:1. The bar charts on the right of Figure 2 quantify these findings for the histological data shown for perivascular fibrosis and interstitial fibrosis at the left of Figure 2. With regard to perivascular fibrosis (bottom bar chart in Figure 2 and Table 2 in Example 2), in control mice fibrotic cardiac tissue accounted for 19% of all tissue analyzed in the untreated group. In contrast, the group treated with a combination of MI and DCI in a respective approximate weight ratio of 40:1 accounted for only 8.52% of all tissue analyzed, corresponding to a decrease of perivascular cardiac fibrosis by about 55% in the treatment group relative to the untreated control. With respect to interstitial fibrosis (top bar chart in Figure 2 and Table 2 in Example 2), interstitial cardiac fibrosis decreased from 11% of total cardiac tissue analyzed in the untreated group to 2.35% of total cardiac tissue analyzed in the group treated with MI and DCI, corresponding to a decrease of interstitial cardiac fibrosis by about 79% in the treatment group relative to the untreated control.

### Detailed description of the invention

The following provides definitions of various terms and expressions used in the present application to describe the invention. It is to be understood that definitions of terms and expressions used in more than one aspect or embodiment of the invention apply equally to any aspect or embodiment of the invention described herein in which those terms and expressions appear. This applies equally regardless of where, i.e. which section, within the present application such terms are defined or discussed.

Further, although the present application mentions discrete embodiments, it is to be understood that any embodiment, and the features therein, may be freely combined with any other embodiment and the features therein, even in the absence of an explicit statement to this effect. Such combinations of one embodiment with another, or of one or more features in any one embodiment with one or more features in any other embodiment, thus belong to the disclosure of the present application as filed as understood by the skilled person. The invention is defined by the claims. Any subject-matter falling outside the scope of the claims is provided for information purposes only.

In this application, the use of the singular (e.g. "a" or "the") may include the plural unless specifically stated otherwise. Further, the use of the term "including" as well as other grammatical forms such as "includes" and "included", is not limiting.

As used herein the term "comprising" has the broad standard meaning "including", "encompassing", or "containing". It includes the explicitly recited element(s), and also allows, but does not require, the presence of other or another element(s) not recited. In addition to this broad meaning, as used herein, the term "comprising" also encompasses the limiting meaning "consisting of", according to which only the explicitly recited element(s), and no other(s) are present. In addition, the term "comprising" also includes the meaning of "consisting essentially of", which means that other element(s) may be present beyond those explicitly recited, provided the additionally present element(s) does not alter the technical effect achieved by the explicitly recited element(s).

As used herein the term "about" when referring to a particular value, e.g. an endpoint or endpoints of a range, encompasses and discloses, in addition to the specifically recited value itself, a certain variation around the specifically recited value. Such a variation may for example arise from normal measurement variability, e.g. in the weighing or apportioning of various substances by methods known to the skilled person. The term "about" shall be understood as encompassing and disclosing a range of variability above and below and indicated specific value, said percentage values being relative to the specific recited value itself, as follows. The term "about" may encompass and disclose variability of ± 5.0%. The term "about" may encompass and disclose variability of ± 4.9%. The term "about" may encompass and disclose variability of ± 4.8%. The term "about" may encompass and disclose variability of ± 4.7%. The term "about" may encompass and disclose variability of ± 4.6%. The term "about" may encompass and disclose variability of ± 4.5%. The term "about" may encompass and disclose variability of ± 4.4%. The term "about" may encompass and disclose variability of ± 4.3%. The term "about" may encompass and disclose variability of ± 4.2%. The term "about" may encompass and disclose variability of ± 4.1%. The term "about" may encompass and disclose variability of ± 4.0%. The term "about" may encompass and disclose variability of ± 3.9%. The term "about" may encompass and disclose variability of ± 3.8%. The term "about" may encompass and disclose variability of ± 3.7%. The term "about" may encompass and disclose variability of ± 3.6%. The term "about" may encompass and disclose variability of ± 3.5%. The term "about" may encompass and disclose variability of ± 3.4%. The term "about" may encompass and disclose variability of ± 3.3%. The term "about" may encompass and disclose variability of ± 3.2%. The term "about" may encompass and disclose variability of ± 3.1%. The term "about" may encompass and disclose variability of ± 3.0%. The term "about" may encompass and disclose variability of ± 2.9%. The term "about" may encompass and disclose variability of ± 2.8%. The term "about" may encompass and disclose variability of ± 2.7%. The term "about" may encompass and disclose variability of ± 2.6%. The term "about" may encompass and disclose variability of ± 2.5%. The term "about" may encompass and disclose variability of ± 2.4%. The term "about" may encompass and disclose variability of ± 2.3%. The term "about" may encompass and disclose variability of ± 2.2%. The term "about" may encompass and disclose variability of ± 2.1%. The term "about" may encompass and disclose variability of ± 2.0%. The term "about" may encompass and disclose variability of ± 1.9%. The term "about" may encompass and disclose variability of ± 1.8%. The term "about" may encompass and disclose variability of ± 1.7%. The term "about" may encompass and disclose variability of ± 1.6%. The term "about" may encompass and disclose variability of ± 1.5%. The term "about" may encompass and disclose variability of ± 1.4%. The term "about" may encompass and disclose variability of ± 1.3%. The term "about" may encompass and disclose variability of ± 1.2%. The term "about" may encompass and disclose variability of ± 1.1%. The term "about" may encompass and disclose variability of ± 1.0%. The term "about" may encompass and disclose variability of ± 0.9%. The term "about" may encompass and disclose variability of ± 0.8%. The term "about" may encompass and disclose variability of ± 0.7%. The term "about" may encompass and disclose variability of ± 0.6%. The term "about" may encompass and disclose variability of ± 0.5%. The term "about" may encompass and disclose variability of ± 0.4%. The term "about" may encompass and disclose variability of ± 0.3%. The term "about" may encompass and disclose variability of ± 0.2%. The term "about" may encompass and disclose variability of 0.1%. The term "about", in reference to the particular recited value, may encompass and disclose that exact particular value itself, irrespective of any explicit mention that this exact particular value is included; even in the absence of an explicit indication that the term "about" includes the particular exact recited value, this exact particular value is still included in the range of variation created by the term "about", and is therefore disclosed. Unless stated otherwise, if the term "about" is recited before the first endpoint of a numerical range, this term refers to both the first endpoint of the range and the second endpoint of the range. For instance, a recited range of "about X to Y" should be read as "about X to about Y". The same applies for a recited range of weight ratios. For instance, a recited range of weight ratios of "about X:Y - A:B" should be read as a weight ratio of "(about X):(about Y) - (about A):(about B)".

Unless stated otherwise, the designation of a range in the present application using a hyphen ("-") separating two bracketing values X and Y, or two bracketing ratios, is to be understood as meaning and disclosing the specified range in which both endpoint values X and Y are included. The same applies to a range expressed as "from X to Y". Accordingly, the expressions of ranges as "X - Y", "of X to Y", "from X to Y", "of X - Y" and "from X - Y" are to be understood equivalently as meaning and disclosing a range encompassing the end value X, all values between X and Y, as well as the end value Y. In contrast, the designation of a range in the present application using the word "between" preceding two bracketing values X and Y, or two bracketing ratios, is to be understood as meaning and disclosing the specified range in which both endpoint values X and Y are excluded, but all values between the specified endpoint values X and Y are included.

As is set out above, the present invention relates to inositol for use in a method of treating or preventing reactive muscle fibrosis in a subject. In a related aspect, the present invention pertains to a composition for use in a method of treating reactive muscle fibrosis in a subject in need or potential need thereof, comprising the step of administering inositol to said subject. In a further related aspect, the present invention pertains to a use of inositol in the manufacture of a medicament for treating or preventing reactive muscle fibrosis in a subject. In some embodiments of the aspects mentioned above, the reactive muscle fibrosis is cardiac fibrosis. In some embodiments, the cardiac fibrosis is perivascular cardiac fibrosis, interstitial cardiac fibrosis or both perivascular cardiac fibrosis and interstitial cardiac fibrosis.

As used herein, the term "subject" refers to a mammal, preferably a human. The subject may be in need of treating reactive muscle fibrosis, e.g. cardiac fibrosis. In such cases, this need will typically have been previously determined by obtaining a diagnosis of reactive muscle fibrosis, in particular a diagnosis of cardiac fibrosis, e.g. a diagnosis of perivascular cardiac fibrosis, a diagnosis of interstitial cardiac fibrosis, or combined diagnoses of both perivascular and interstitial cardiac fibroses. In such cases, in which a diagnosis or diagnoses of a pathological condition or pathological conditions already exists, the invention as set out herein may be implemented to treat existing pathology (e.g. reactive fibrosis, which is reparable), while simultaneously preventing the development of further pathology (e.g. reactive fibrosis and/or reparative fibrosis). It is however also contemplated that the subject matter of the invention as set out herein can be applied to subjects suspected of having a need thereof. Such subjects will typically not have been previously diagnosed for reactive muscle fibrosis, e.g. cardiac fibrosis, e.g. perivascular cardiac fibrosis or interstitial cardiac fibrosis, yet such subjects may be at risk for developing this condition. For instance, overweight or obese subjects, subjects with a positive diagnosis for a pre-diabetic or diabetic state, and subjects affected by MetS or more generally by dysmetabolisms are at risk for developing reactive muscle fibrosis, in particular cardiac fibrosis. The invention described herein this also envisions administering inositol(s) to a subject which has not been previously diagnosed for reactive muscle fibrosis, for example cardiac fibrosis, with the aim of inhibiting the development of (e.g. further) reactive muscle fibrosis, in particular cardiac fibrosis.

Accordingly, as used herein, the term "treat" or grammatically related variants thereof such as "treatment", "treating" etc. means the amelioration, even temporarily, encompassing but not requiring the complete abolishment of a pathological state. In the broadest sense, treatment of reactive muscle fibrosis, by the medical uses and methods of treatment herein means achieving at least a partial reversion of dysfunctional fibrotic tissue to its previously functional state. In particular, treatment in the present sense encompasses restoration of proper organ architecture and function in organs in which fibrotic tissue has developed. For instance, in the case the organ is the heart, and the reactive muscle fibrosis is cardiac fibrosis, treatment would encompass reduction of fibroblasts and extracellular matrix (ECM) protein in fibrotic tissue to restore functional cardiac tissue.

As used herein, the term "reactive muscle fibrosis" refers to a condition in which healthy muscle tissue is remodeled due to a chronic metabolic insult to contain increased amounts of fibroblasts and ECM proteins, leading to an altered, pathological function. In certain embodiments herein, the reactive muscle fibrosis is cardiac fibrosis, in which the muscle tissue is cardiac tissue. It is understood that "cardiac fibrosis" as used and disclosed herein denotes a subform of "reactive muscle fibrosis". Accordingly, the term "cardiac fibrosis" is to be understood as meaning and disclosing "reactive cardiac fibrosis" even if the term "reactive" is not recited as modifying "cardiac fibrosis". In certain embodiments herein, the cardiac fibrosis is perivascular cardiac fibrosis or interstitial cardiac fibrosis. In certain embodiments, the cardiac fibrosis is a combination of perivascular cardiac fibrosis and interstitial cardiac fibrosis.

As used herein, the term "prevent" or grammatically related variants thereof such as "prevention" refer to scenarios in which the medical uses and methods of treatment described herein are applied to avert the possible, suspected or expected occurrence of reactive muscle fibrosis such as cardiac fibrosis. Such suspicion that reactive muscle fibrosis such as cardiac fibrosis may derive (even in the absence of a corresponding diagnosis) from the subject history, for example if the subject had previously suffered from reactive muscle fibrosis which had been previously treated. In such a scenario, there may exist a justified suspicion that previously treated reactive muscle fibrosis, e.g. cardiac fibrosis, may reoccur.

As used herein, the term "inositol" encompasses and discloses the substance having chemical formula C₆H₁₂O₆ and a structure according to any of the 9 known isomers of inositol, that is:

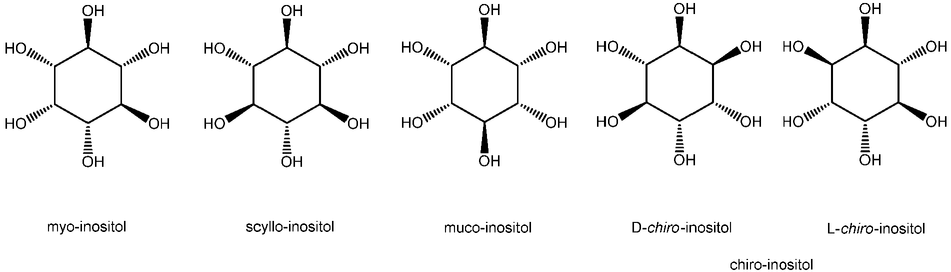

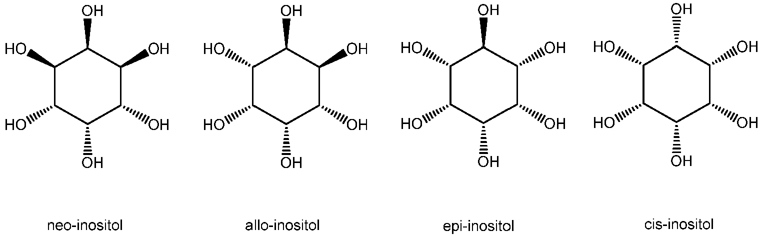

In a preferred embodiment of the present invention, the inositol is myoinositol (MI). In a further preferred embodiment of the present invention, the inositol is a combination of MI and at least one isomer of inositol other than MI (hereinafter "non-MI inositol"). In a further preferred embodiment of the present invention, the at least one non-MI inositol includes D-chiro-inositol (DCI). In another preferred embodiment of the present invention, the inositol comprises a combination of MI and DCI in any respective weight ratio.

In one embodiment the respective weight ratio of MI to DCI (MI:DCI) in a combination thereof may advantageously be from about 100:1 to about 10:1. In a further embodiment the respective weight ratio of MI:DCI in a combination thereof may advantageously be between 100:1 and 10:1. In a further embodiment the respective weight ratio of MI:DCI in a combination thereof may advantageously be from about 80:1 to about 20:1. In a further embodiment the respective weight ratio of MI:DCI in a combination thereof may advantageously be between 80:1 and 20:1. In a further embodiment the respective weight ratio of MI:DCI in a combination thereof may advantageously be from about 60:1 to about 30:1. In a further embodiment the respective weight ratio of MI:DCI in a combination thereof may advantageously be between 60:1 and 30:1. In a further embodiment the respective weight ratio of MI:DCI in a combination thereof may advantageously be from about 50:1 to about 35:1. In a further embodiment the respective weight ratio of MI:DCI in a combination thereof may advantageously be between 50:1 and 35:1. In an further especially preferred embodiment the respective weight ratio of MI:DCI in a combination thereof may advantageously be about 40:1 or about 10:1. In a further especially preferred embodiment the respective weight ratio of MI:DCI any combination thereof may advantageously be 40:1 or 10:1.

As used herein, the term "weight ratio" as applies to the relative amounts of two substances in e.g. composition, denotes the ratio of the weight of one compound relative to the weight of another compound in the respective composition. In particular, the weight ratio does not take account of the weight of any fillers, excipients, diluents, etc. which, in addition to the substances for which the weight ratio is specified, may also be present in the combination or composition. As a particular nonbinding example, a composition comprising a weight ratio of MI:DCI of about 40:1 denotes a composition in which, by weight, the amount of MI exceeds that of DCI by a factor of about 40, regardless of the weight of any additional fillers, excipients, diluents, etc. which may be present in the composition. In the event a combination of inositols is anything other than a combination in solid form, a specified weight ratio pertains to the weight amounts of components in that combination in their corresponding solid form, prior to solubilization, emulsification, suspension, etc. in the subject combination. It is understood that in the case of a mixture of different types of inositol, the weight of "inositol", e.g. in the sense of any of the weight ratios mentioned herein, will be the sum of all types of inositol present.

As used herein, the term "combination" refers to the coupling of MI and at least one non-MI inositol for the purpose of their co-administration to a subject within a given treatment regimen. A given treatment regimen may comprise multiple, repeated co-administrations of inositol and the at least one non-MI inositol over a predetermined time course, e.g. over 1 month, over 2 months, over 3 months, over 4 months or over 5 months or more, a time course of at least 3 months being preferred. The co-administration of MI and at least one non-MI inositol may be repeated multiple times daily, for example once, twice, 3 times, 4 times, 5 times or more, a repetition about twice daily being preferred. That is, the co-administration of MI and at least one non-MI inositol is repeated over a predetermined length of time, and the sum of the instances of co-administration over this predetermined length of time constitutes the prophylactic or therapeutic regimen.

The coupling of MI and at least one non-MI inositol meant by a "combination" of these two substances need not be physical, nor need it imply simultaneity of administration. Reference to a "combination" of MI and at least one non-MI inositol therefore encompasses and discloses multiple possibilities regarding the route and timing of administration of the respective substances. Encompassed and disclosed in the meaning of "combination" is for example the coupling of MI and at least one non-MI inositol for simultaneous administration by the same route, simultaneous administration by different routes, chronologically staggered (i.e. non-simultaneous) administration by the same route, or chronologically staggered (i.e. non-simultaneous) administration by different routes. Any of the administration routes described herein may be combined in any way, although it will generally be preferable that the route will be oral. Especially preferable is simultaneous administration of MI and at least one non-MI inositol by the oral route, e.g. when MI and at least one non-MI inositol are present in a composition, e.g. in one of the inventive compositions set out herein, e.g. in the form of a tablet, hard capsule, soft gel capsule, or powder, e.g. in the form of a sachet, for ingestion.

Simultaneous administration of MI and at least one non-MI inositol by the same route might for example take the form of MI and at least one non-MI inositol being comprised in the same physical composition, that composition being administered to, e.g. ingested by, the subject so that the MI and at least one non-MI inositol enter the body by the same route, e.g. by the oral route, at the same time. Coupling of MI and at least one non-MI inositol for staggered (i.e. non-simultaneous) administration within a given co-administration is however also possible, and is also encompassed and disclosed in the term "combination". The order of administration of MI and at least one non-MI inositol is not particularly important; the chronologically staggered (i.e. non-simultaneous) administration of MI and at least one non-MI inositol might encompass prior administration of MI and subsequent administration of at least one non-Ml inositol, or prior administration of at least one non-MI inositol and subsequent administration of Ml. For example, staggered (i.e. non-simultaneous) administration of MI and at least one non-MI inositol by the same route may take the form of initial oral administration of at least one non-Ml inositol, followed by oral administration of MI; coupling of MI and at least one non-MI inositol in this way falls within the meaning of, and is disclosed by, "combination" as used herein. Conversely, staggered (i.e. non-simultaneous) administration of MI and at least one non-MI inositol by different routes might for instance take the form of initial oral administration of at least one non-Ml inositol, followed by administration of MI by e.g. suppository; coupling of MI and at least one non-MI inositol in such ways also falls within the meaning of, and is disclosed by, "combination" as used herein.

As used herein, the term "non-simultaneously" means that the different isomers of inositol, e.g. MI and at least one non-MI inositol, e.g. DCI, are administered in a chronologically staggered manner, i.e. at different times. The administration refers to a respective co-administration of such inositol(s) within a broader regimen of treatment or prophylaxis. For instance, one embodiment entails administering MI to the subject prior to administering the at least one non-Ml inositol to the subject. Another embodiment entails administering MI to the subject following administering the at least one non-Ml inositol to the subject. It is understood that such non-simultaneous administration denotes any respective instance of coadministration of differing inositol isomers, e.g. MI and at least one non-MI inositol, e.g. DCI, within the broader context of the overall treatment regimen. As mentioned herein, regardless of the order of administration, that is e.g. MI first and non-MI inositol, e.g. DCI second, or *vice versa,* the respective combined administration of inositol isomers may be by the same or different routes.

In a further embodiment the various inositol isomers are administered to the subject simultaneously. As mentioned above, the simultaneous administration of various inositol isomers can be effected by the same or different routes. Most typically, it will be most advantageous and convenient to effect the simultaneous administration of multiple inositol isomers by the same route, preferably by an oral administration route. This will most often be accomplished by combining the inositol isomers to be administered in the same composition, for example in the form of a tablet, including but not limited to an effervescent tablet, a powder (especially presented in the form of a sachet), a hard capsule, a soft gel capsule, a syrup, a cachet, a troche, or a lozenge.

There are no particular restrictions on the duration of time between administration of MI and at least one non-MI inositol in the event the co-administration is chronologically staggered, i.e. non-simultaneous, however it will generally be most effective and convenient when any interim between administering MI and at least one non-MI inositol is brief, approaching simultaneity, e.g. on the order of minutes. In some cases such an interim may extend up to about an hour or several hours between respective administrations. In some cases, then, in the event that the co-administration of MI and at least one non-MI inositol is chronologically staggered, the interim between administration in some instances may be as long as 12 hours. However, even in the event the MI and at least one non-MI inositol are administered in a chronologically staggered manner, whether by the same or different routes, the term "combination" means that a given co-administration of both Ml and at least one non-MI inositol within any regimen will have been completed, i.e. both substances will have been administered, by the time the respective next co-administration of MI and at least one non-MI inositol (which itself may again be either simultaneous or chronologically staggered by the same or different routes in a manner corresponding to are different from the chronological staggering and routes of the previous co-administration) within the same regimen ensues.

In the event that Ml and the at least one non-MI inositol are administered to the subject simultaneously, the MI and at least one non-MI inositol are preferably present in a composition comprising the MI and at least one non-MI inositol.

As used herein, the term "composition" encompasses and discloses any physical entity comprising or consisting of or consisting essentially of the respective recited substances, e.g comprising or consisting of or consisting essentially of the MI and at least one non-MI inositol. The physical form of the composition is not restricted. For example, the term "composition" encompasses and discloses a powder in which the recited substances are each present in powder form. As a further example, the term "composition" also encompasses and discloses a liquid solution in which the recited substances are present in solubilized form. As a further example, the term "composition" also encompasses and discloses an emulsion in which the recited substances are present. As a further example, the term "composition" also encompasses and discloses a suspension in which the recited substances are present. As a further example, the term "composition" also encompasses and discloses mixtures in which the MI is in one form, e.g. a solid such as a powder, while the non-MI inositol is in another form, e.g. a liquid. In particular, the term "composition" may be a "pharmaceutical composition" as defined herein below, and may be formulated for a desired route of administration. As used and disclosed herein, the term "composition" may also be a composition suitable for oral delivery, e.g. in the form of a tablet, including but not limited to an effervescent tablet or a multilayer tablet, a powder, e.g. in the form of a sachet, a hard capsule, a soft gel capsule, a syrup, a cachet, a troche, or a lozenge, a pastille, e.g. a gummy pastille, a salve, or a liquid preparation. In certain especially preferred embodiments of the invention, the "composition" comprising inositol, e.g. Ml, alone or in combination with at least one non-Ml inositol, may be in the form of a soft gel capsule. In certain other especially preferred embodiments of the invention, the "composition" comprising inositol, e.g. Ml, alone or in combination with at least one non-Ml inositol, maybe in the form of a powder. The term "composition" may also be a composition suitable for delivery by a non-oral route, for example in the form of a suppository, a tablet, a hard capsule, a soft gel capsule, a cream, a gel, a patch or a liquid. Further dosage forms of the composition as well as referred routes of administration are set out herein below.

In the medical uses and treatment methods described herein, it is contemplated that the inositol may be administered in combination with one or more additional substances.

For example, the inositol may be administered in combination with a source of folate. The inclusion of a source of folate for administration in combination with the inositol according to the medical uses and methods of treatment set out herein may be especially advantageous in case of cardiac fibrosis. Folate intake reduces homocysteine levels, and hyperhomocysteinemia is considered an independent risk factor for cardiovascular disease. As used herein, the term a "source of folate" or grammatically related terms such as "folate source" means any compound which, when introduced into the body, provides a source of folate to the body. One such source of folate is folic acid having the art-accepted definition of the compound of chemical formula C₁₉H₁₉N₇O₆ designated *N*-(4-f[(2-amino-4-oxo-1,4-dihydropteridin-6-yl)methyl]amino}benzoyl)-L-glutamic acid (IUPAC designation (2S)-2-[[4-[(2-amino-4-oxo-1H-pteridin-6-yl)methylamino]benzoyl]amino]pentanedioic acid), and having the chemical structure:

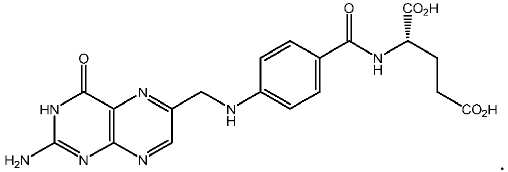

A source of folate may also be folate itself, e.g. in one of the salt forms of deprotonated folic acid. Another suitable source of folate is 5-methyltetrahydrofolate.

In the event the inositol is administered in combination with a source of folate, e.g. folate, the inositol: folate source weight ratio may be about 1 : 0.004. It is understood that the specific type of inositol in the inventive composition may be any of the inositol isomers or mixtures thereof set out hereinabove. For instance, one embodiment of the medical uses and methods of treatment described herein may entail combined administration of about 2000 mg inositol and about 200 µg folic acid. A further embodiment of the medical uses and methods of treatment described herein may entail combined administration of about 2000 mg myoinositol and about 200 µg folic acid. A further embodiment of the medical uses and methods of treatment described herein may entail combined administration of a combination of MI and DCI in any weight ratio, in particular a respective MI:DCI weight ratio of about 40:1 and about 200 µg folic acid.

As another example, the inositol in the medical uses and methods of treatment described herein may also be administered in combination with one or more polyphenols. The inclusion of one or more polyphenols for administration in combination with the inositol according to the medical uses and methods of treatment set out herein may be especially advantageous if the reactive muscle fibrosis to be prevented or treated is cardiac fibrosis. As example, polyphenols can derive from cocoa, olive oil, grape and pomegranate. Cocoa polyphenols are known to have a positive effect on cardiovascular disease; olive oil polyphenols, such as hydroxityrosol, are known for potent anti-oxidant and anti-inflammatory effects, among others: the importance in protection of low-density lipoproteins and consequently its implication in the reduction of cardiovascular disease risk has been reported. Therefore, in the event that the reactive muscle fibrosis is cardiac fibrosis, it is to be expected that other cardiac pathologies besides cardiac fibrosis may exist. In such scenarios, inclusion of one or more polyphenols in the administration in combination with inositol may represent an efficient way of achieving parallel ameliorative effects.

As another example, the inositol may be administered in combination with one or more carotenoids. The inclusion of one or more carotenoid for administration in combination with the inositol according to the medical uses and methods of treatment set out herein may be especially advantageous if the reactive muscle fibrosis to be prevented or treated is cardiac fibrosis. Carotenoids may reduce the risk of cardiovascular diseases by preventing the oxidation of cholesterol in arteries. A possible carotenoid may be fucoxanthin, a specific carotenoid found in brown seaweed, whose intake has been shown to reduce the body weight.

As described above, the present invention relates to the treatment and prevention of reactive muscle fibrosis, in particular cardiac fibrosis. Specific types of cardiac fibrosis treatable by the present invention include perivascular cardiac fibrosis and interstitial cardiac fibrosis. A composition comprising inositol, for example MI alone or in combination with at least one non-MI inositol, e.g. DCI, may advantageously be provided in a form suitable to allow the inositol or inositol combination to be taken up into the body. As such, a composition in which the inositol or inositol combination is administered will thus generally be manufactured according to processes established for the manufacture of pharmaceutical products.

Accordingly, in one embodiment, the inventive composition is a pharmaceutical composition. The compositions of the invention, including pharmaceutical compositions, may further comprise at least one pharmaceutically acceptable ingredient. While the inventive composition, including the pharmaceutical composition, may itself be administered to a subject, it will be understood that adding one or more pharmaceutically acceptable ingredients beyond the inositol, e.g. MI alone or in combination with at least one non-Ml inositol, may be advantageous in rendering the composition more suitable for direct administration to a subject by a given predetermined route. Accordingly, the "pharmaceutical composition", may be formulated to comprise, in addition to inositol(s), a pharmaceutically acceptable ingredient which renders the composition more suitable or especially suitable for direct administration to a subject by a given route, without further workup. This suitability may pertain to a number of different administration routes, including oral, parenteral, transmucosal, vaginal or perivaginal, topical, transdermal or intravesical, as further set out in the following. Where present, and by way of non-limiting illustration, such ingredients as well as their advantageous impact on the suitability of the inventive composition for different routes of administration are set out below.

### a) Formulation for suitability for oral administration

A composition suitable for oral administration may be prepared, packaged, or sold in the form of a discrete solid dose unit including a sachet (e.g. a powder in a sachet), a tablet, a hard or soft capsule, a cachet, a troche, or a lozenge, each containing a predetermined amount of inositol(s) in a ratio as specified herein. Other formulations suitable for oral administration include a powdered or granular formulation, an aqueous or oily suspension, an aqueous or oily solution, or an emulsion. As used herein, an "oily" liquid comprises a carbon-containing liquid molecule that exhibits a less polar character than water.

It is especially preferred that the discrete solid dose unit of the inventive composition is in the form of a powder, especially presented in the form of a sachet. As used herein, the term "sachet" refers to a sealed pouch containing the inventive composition. The pouch may be made of paper, wax paper, plasticized paper, or a combination of paper and foil. The material out of which the sachet is fashioned is preferably impervious to ambient moisture and other potential atmospheric contaminants so that, by sealing said sachet, the inventive composition in the form of a powder contained therein remains in a free flowing form until use.

A tablet comprising the inositol(s), e.g. MI and at least one non-MI inositol in any of the above weight ratios, may for example be made by compressing or molding the inositol(s) with one or more additional ingredients. Compressed tablets may be prepared by compressing, in a suitable device, inositol(s) in a free flowing form such as a powder or granular preparation, optionally mixed with one or more of a binder, a lubricant, an excipient, a surface active agent, and a dispersing agent. Molded tablets may for example be made by molding, in a suitable device, inositol(s), a pharmaceutically acceptable carrier, and at least sufficient liquid to moisten the mixture.

Pharmaceutically acceptable excipients used in the manufacture of tablets include, but are not limited to, inert diluents, granulating and disintegrating agents, binding agents, and lubricating agents. Known dispersing agents include, but are not limited to, potato starch and sodium starch glycolate. Known surface active agents include, but are not limited to, sodium lauryl sulfate. Known diluents include, but are not limited to, calcium carbonate, sodium carbonate, lactose, microcrystalline cellulose, calcium phosphate, calcium hydrogen phosphate, and sodium phosphate. Known granulating and disintegrating agents include, but are not limited to, corn starch and alginic acid. Known binding agents include, but are not limited to, gelatin, acacia, pre-gelatinized maize starch, polyvinylpyrrolidone, and hydroxypropyl methylcellulose. Known lubricating agents include, but are not limited to, magnesium stearate, stearic acid, silica, and talc.

Tablets may be non-coated or they may be coated using known methods to achieve delayed disintegration in the gastrointestinal tract of a subject, thereby providing sustained release and absorption of the inositol(s). Following ingestion, a composition will advantageously be released prior to passing to the small intestine, where primary inositol resorption takes place. By way of example, a material such as glyceryl monostearate or glyceryl distearate may be used to coat tablets. Tablets may further comprise a sweetening agent, a flavoring agent, a coloring agent, a preservative, or some combination of these in order to provide pharmaceutically attractive and palatable preparation.

Hard capsules comprising the inositol(s) may be made using a physiologically degradable composition, such as gelatin or cellulose derivatives. Such hard capsules comprise the inositol(s), and may further comprise additional ingredients including, for example, an inert solid diluent such as calcium carbonate, calcium phosphate, or kaolin.

Soft gelatin capsules comprising the inositol(s) may be made using a physiologically degradable composition, such as gelatin combined with a plasticizer (i.e. glycerol) as basic component of the soft gelatin shell. Soft gelatin capsules may contain a liquid or semisolid solution, suspension, or microemulsion preconcentrate. The soft capsule filling comprises the inositol(s), which may be mixed with water or an oil medium such as peanut oil, liquid paraffin, olive oil, soybean oil, sunflower oil, a lecithin such as for example soy lecithin or sunflower lecithin, medium chain triglycerides, polyglycerol oleate, beeswax, mono- and diglycerides of fatty acids, or combinations of any of the above.

Liquid formulations that are especially suitable for oral administration may be prepared, packaged, and sold either in liquid form or in the form of a dry product intended for reconstitution with water or another suitable vehicle prior to ingestion.

Liquid suspensions may be prepared using conventional methods to achieve suspension of the active ingredient(s) in an aqueous or oily vehicle. Aqueous vehicles include, for example, water and isotonic saline. Oily vehicles include, for example, almond oil, oily esters, ethyl alcohol, vegetable oils such as arachis, olive, sesame, or coconut oil, fractionated vegetable oils, and mineral oils such as liquid paraffin. Liquid suspensions may further comprise one or more additional ingredients including, but not limited to, suspending agents, dispersing or wetting agents, emulsifying agents, demulcents, preservatives, buffers, salts, flavorings, coloring agents, and sweetening agents. Oily suspensions may further comprise a thickening agent. Known suspending agents include, but are not limited to, sorbitol syrup, hydrogenated edible fats, sodium alginate, polyvinylpyrrolidone, gum tragacanth, gum acacia, and cellulose derivatives such as sodium carboxymethylcellulose, methylcellulose, hydroxypropyl methylcellulose. Known dispersing or wetting agents include, but are not limited to, naturally occurring phosphatides such as lecithin, condensation products of an alkylene oxide with a fatty acid, with a long chain aliphatic alcohol, with a partial ester derived from a fatty acid and a hexitol, or with a partial ester derived from a fatty acid and a hexitol anhydride (e.g., polyoxyethylene stearate, heptadecaethyleneoxycetanol, polyoxyethylene sorbitol monooleate, and polyoxyethylene sorbitan monooleate, respectively). Known emulsifying agents include, but are not limited to, lecithin and acacia. Known preservatives include, but are not limited to, methyl, ethyl, or n-propyl para-hydroxybenzoates, ascorbic acid, and sorbic acid. Known sweetening agents include, for example, glycerol, propylene glycol, sorbitol, sucrose, and saccharin. Known thickening agents for oily suspensions include, for example, beeswax, hard paraffin, and cetyl alcohol.

Powdered and granular formulations of a composition suitable for application in the present invention, e.g. a pharmaceutical composition, may be prepared using known methods. Such formulations may be administered directly to a subject, used, for example, to form sachet or tablets, to fill capsules, or to prepare an aqueous or oily suspension or solution by addition of an aqueous or oily vehicle thereto. Each of these formulations may further comprise one or more of dispersing or wetting agent, a suspending agent, and a preservative. Additional excipients, such as fillers and sweetening, flavoring, or coloring agents, may also be included in these formulations.

A composition may also be prepared, packaged, or sold in the form of oil-in-water emulsion or a water-in-oil emulsion. The oily phase may be a vegetable oil such as olive or arachis oil, a mineral oil such as liquid paraffin, or a combination of these. Such compositions may further comprise one or more emulsifying agents such as naturally occurring gums such as gum acacia or gum tragacanth, naturally occurring phosphatides such as soybean or lecithin phosphatide, esters or partial esters derived from combinations of fatty acids and hexitol anhydrides such as sorbitan monooleate, and condensation products of such partial esters with ethylene oxide such as polyoxyethylene sorbitan monooleate. These emulsions may also contain additional ingredients including, for example, sweetening or flavoring agents.

Methods for impregnating or coating a material with a chemical composition are known in the art, and include, but are not limited to methods of depositing or binding a chemical composition onto a surface, methods of incorporating a chemical composition into the structure of a material during the synthesis of the material (e.g. such as with a physiologically degradable material), and methods of absorbing an aqueous or oily solution or suspension into an absorbent material, with or without subsequent drying.

### b) Formulation for suitability for parenteral administration

For parenteral administration, a composition comprising inositol(s) may be formulated for injection or infusion, for example, intravenous, intramuscular or subcutaneous injection or infusion, or for administration in a bolus dose and/or continuous infusion. Suspensions, solutions or emulsions in an oily or aqueous vehicle, optionally containing other agents such as suspending, stabilizing and/or dispersing agents such as those mentioned above, may be used.

A composition comprising inositol(s) may be rendered especially suitable for parenteral administration by formulation with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampoules, crushable or otherwise, or in multi-dose containers containing a preservative. Compositions especially suitable for parenteral administration include, but are not limited to, suspensions, solutions, emulsions in oily or aqueous vehicles, pastes, and implantable sustained-release or biodegradable formulations. Such compositions may further comprise one or more additional ingredients including, but not limited to, suspending, stabilizing, or dispersing agents. In one embodiment of a composition which is especially suitable for parenteral administration, the active ingredient is provided in dry (e.g. powder or granular) form for reconstitution with a suitable vehicle (e.g. sterile pyrogen free water) prior to parenteral administration of the reconstituted composition.

A composition suitable for application in the present invention may be prepared, packaged, or sold in the form of a sterile injectable aqueous or oily suspension or solution. This suspension or solution may be formulated according to the known art, and may comprise, in addition to the active ingredient(s), additional ingredients such as the dispersing agents, wetting agents, or suspending agents described herein. Such sterile injectable compositions may be prepared using a non toxic, parenterally acceptable diluent or solvent, such as water or 1,3-butanediol, for example. Other acceptable diluents and solvents include, but are not limited to, Ringer's solution, isotonic sodium chloride solution, and fixed oils such as synthetic mono- or diglycerides. Other usual parentally-administrable formulations include those that comprise the active ingredient in microcrystalline form, in a liposomal preparation, or as a component of a biodegradable polymer system. Compositions for sustained release or implantation may comprise pharmaceutically acceptable polymeric or hydrophobic materials such as an emulsion, an ion exchange resin, a sparingly soluble polymer, or a sparingly soluble salt.

### c) Formulation for suitability for transmucosal administration

A composition comprising inositol(s), e.g. MI and at least one non-Ml inositol, may be formulated to be suitable for transmucosal administration. The formulation may include any substances or dosage unit suitable for application to mucosal tissue. For example, the selected active agent may be administered to the buccal mucosa in an adhesive tablet or patch, sublingually administered by placing a solid dosage form under the tongue, lingually administered by placing a solid dosage form on the tongue, administered nasally as droplets or a nasal spray, a non-aerosol liquid formulation, or a dry powder, placed within or near the rectum ("transrectal" formulations), or administered to the urethra as a suppository, ointment, or the like.

### d) Formulation for suitability for vaginal or perivaginal administration

A composition comprising inositol(s) may also be formulated to be especially suitable for vaginal or perivaginal administration. Suitable dosage forms to this end may include vaginal suppositories, creams, ointments, liquid formulations, pessaries, tampons, gels, pastes, foams or sprays. The suppository, cream, ointment, liquid formulation, pessary, tampon, gel, paste, foam or spray for vaginal or perivaginal delivery comprises a therapeutically effective amount of the selected active agent(s) and one or more conventional nontoxic carriers suitable for vaginal or perivaginal drug administration. The vaginal or perivaginal forms of the present invention may be manufactured using conventional processes as for example disclosed in Remington: The Science and Practice of Pharmacy, supra. The vaginal or perivaginal dosage unit may be fabricated to disintegrate rapidly or over a period of several hours. The time period for complete disintegration may be in the range of from about 10 minutes to about 6 hours, e.g., less than about 3 hours.

### e) Formulation for suitability for topical formulations

A composition comprising inositol(s) may also be formulated to be especially suitable for topical administration. Suitable dosage forms to this end may include any form suitable for application to the body surface, and may comprise, for example, an ointment, cream, gel, lotion, solution, paste or the like, and/or may be prepared so as to contain liposomes, micelles, and/or microspheres. In certain embodiments, topical formulations herein are ointments, creams and gels.

### f) Formulation for suitability for transdermal administration

A composition comprising inositol(s) may also be formulated to be especially suitable for transdermal administration. As known to one skilled in the art, transdermal administration involves the delivery of pharmaceutical compounds via percutaneous passage of the compound into the systemic circulation of the patient. This can be affected e.g. by transdermal patches or iontophoresis devices. Other components besides inositol and alpha lactalbumin may be incorporated into the transdermal patches as well. For example, compositions and/or transdermal patches may be formulated with one or more preservatives or bacteriostatic agents including, but not limited to, methyl hydroxybenzoate, propyl hydroxybenzoate, chlorocresol, benzalkonium chloride, and the like. Dosage forms of the inventive composition for topical administration of the inositol(s) may include creams, sprays, lotions, gels, ointments, eye drops, nose drops, ear drops, and the like. In such dosage forms, the ingredients of the inventive composition may be mixed to form a white, smooth, homogeneous, opaque cream or lotion with, for example, benzyl alcohol 1% or 2% (wt/wt) as a preservative, emulsifying wax, glycerin, isopropyl palmitate, lactic acid, purified water and sorbitol solution. In addition, the compositions may contain polyethylene glycol 400. They may be mixed to form ointments with, for example, benzyl alcohol 2% (wt/wt) as preservative, white petrolatum, emulsifying wax, and tenox II (butylated hydroxyanisole, propyl gallate, citric acid, propylene glycol). Woven pads or rolls of bandaging material, e.g., gauze, may be impregnated with the compositions in solution, lotion, cream, ointment or other such form may also be used for topical application. The compositions may also be applied topically using a transdermal system, such as one of an acrylic-based polymer adhesive with a resinous crosslinking agent impregnated with the composition and laminated to an impermeable backing.

Examples of suitable skin contact adhesive materials include, but are not limited to, polyethylenes, polysiloxanes, polyisobutylenes, polyacrylates, polyurethanes, and the like. Alternatively, the drug-containing reservoir and skin contact adhesive are separate and distinct layers, with the adhesive underlying the reservoir that, in this case, may be either a polymeric matrix as described above, or be a liquid or hydrogel reservoir, or take some other form.

### g) Formulation for suitability for intravesical administration

The term intravesical administration is used herein in its conventional sense to mean delivery of a drug directly into the bladder. Suitable methods for intravesical administration may be found, for example, in US Patent Nos. 6,207,180 and 6,039,967.

The inositol or inositol combination may also be administered according to the medical uses and methods of treatment described herein in the form of a food or drink product comprising the inositol or inositol combination. The inositol may be any of the inositol or inositol mixtures set out hereinabove, in particular Ml, DCI or a mixture of MI and DCI in any weight ratio, in particular in a respective MI:DCI weight ratio of about 40:1.

As used herein, the term "food product" refers to an ingestible substance which, at the temperature it is properly stored and ingested, is in a solid or semi solid form, and which will be chewed prior to swallowing. As used herein, the term "drink product" refers to an ingestible substance which, at the temperature it is properly stored and ingested, is in a free-flowing liquid form, and which will not be chewed prior to swallowing.

The food product may in principle be any food product which has been processed to comprise the inositol or inositol combination to be administered. In the event an inositol combination is to be administered, the food product will comprise the relevant inositols in the intended weight ratio, such as any one of the weight ratios for various inositols set out herein. Examples of such food products include a food bar, such as a chocolate bar, granola bar, ice cream bar or energy bar; chewing gum; candy; a breath mint; yogurt; an edible gel; a ready-made meal, for example a freeze-dried ready-made meal; a spread; a pudding; or a processed fruit product such as a fruit rollup or a fruit stick. Examples of such drink products include fruit juice or fruit juice-containing drinks, dairy product drinks, e.g. milk-containing drinks or buttermilk-containing drinks, whey-containing drinks and yogurt-containing drinks, energy drinks, soft drinks, flavored water drinks etc.

The Applicant has found that provision of a certain minimum amount of inositol in the inventive composition can be advantageous in ensuring that the desired therapeutic or prophylactic effect can be achieved in a reasonable number of administrations. Typically, successful treatment or prevention of reactive muscle fibrosis, especially cardiac fibrosis, in particular perivascular cardiac fibrosis, interstitial cardiac fibrosis or both perivascular cardiac fibrosis and interstitial cardiac fibrosis, can be achieved by a daily administration of a total of as much as about 6000 mg total inositol(s), or about 4000 mg total inositol(s). Of course, varying numbers of combined administrations of inositol(s) may be necessary to achieve a given target daily administration of such inositol(s), depending on how much of the inositol(s) is administered in any one instance. However, from the standpoint of the impact of any such repeated administration on patient quality of life, it can be advantageous to ensure a minimum amount of inositol(s) in the composition so as to correspondingly decrease the number of daily administrations needed to reach a given target amount of such inositol(s), e.g. the target amount of inositol(s) indicated above.

Therefore, one embodiment of the medical uses and treatment methods set out herein may involve administering as much as about 6000 mg of total inositol(s) in one combined administration to the subject. Another embodiment of the medical uses and treatment methods set out herein may involve administering about 500 - 4000 mg of total inositol(s) in one combined administration to the subject. Another embodiment of the medical uses and treatment methods set out herein may involve administering about 1000 - 2000 mg of total inositol(s) in one combined administration to the subject. Another embodiment of the medical uses and treatment methods set out herein may involve administering about 2050 mg of total inositol(s) to the subject in one combined administration (e.g. in the form of a powder, e.g. provided in a sachet, for example made up of about 2000 mg MI and about 50 mg DCI). Another embodiment of the medical uses and treatment methods set out herein may involve administering about 560 mg total inositol(s) to the subject in one combined administration. For instance, such medical uses and treatment methods may involve administering 563.8 mg of total inositol(s) to the subject in one combined administration (e.g. in the form of a soft gel capsule for example made up of 550 mg Ml and 13.8 mg DCI).

One embodiment of the medical uses and treatment methods set out herein may involve administering about 1000 - 2000 mg of Ml, or between 1000 and 2000 mg of MI in any one administration, e.g. in any one combined administration, to the subject. Another embodiment of the medical uses and treatment methods set out herein may involve administering about 2000 mg of MI in any one administration, e.g. in any one combined administration, to the subject. Another embodiment of the medical uses and treatment methods set out herein may involve administering about 600 mg of MI in any one administration, e.g. in any one combined administration, to the subject. Another embodiment of the medical uses and treatment methods set out herein may involve administering about 550 mg of MI in any one administration, e.g. in any one combined administration, to the subject. Another embodiment of the medical uses and treatment methods set out herein may involve administering about 5 - 100 mg of DCI, or between 5 and 100 mg of DCI in any one administration, e.g in any one combined administration, to the subject. Another embodiment of the medical uses and treatment methods set out herein may involve administering about 10 - 50 mg of DCI, or between 10 and 50 mg of DCI in any one administration, e.g in any one combined administration, to the subject. Another embodiment of the medical uses and treatment methods set out herein may involve administering about 50 mg of DCI in any one administration, e.g. in any one combined administration, to the subject. Another embodiment of the medical uses and treatment methods set out herein may involve administering about 13.8 mg of DCI in any one administration, e.g. in any one combined administration, to the subject.

In some embodiments, certain specified amounts of inositol(s) may be especially preferable for certain dosage forms. In one embodiment, if the inositol or inositol combination is to be provided for administration to the subject in powder form, e.g. in a sachet, the inositol may comprise or consist of about 2000 mg MI, and this may be administered to the subject. In a related embodiment, the inositol combination may be provided for administration to the subject in powder form, e.g. in a sachet, and the powder composition may comprise about 2000 mg MI and about 50 mg DCI, and this may be administered to the subject. Alternatively, in one embodiment, if the inositol or inositol combination is to be provided for administration to the subject in the form of a soft gel capsule, the inositol may comprise or consist of about 600 mg MI, and this may be administered to the subject. In a related embodiment, the inositol combination may be provided for administration to the subject in the form of a soft gel capsule, and the inositol combination in the soft gel capsule may comprise 550 mg Ml and 13.8 mg DCI, and this may be administered to the subject. In general, it can be advantageous to administer an inositol combination to the subject in powder form, in which the powdered inositol composition comprises MI and DCI in a respective MI:DCI weight ratio of about 40:1, which is about the natural ratio of MI:DCI in the human body. In general, it can also be advantageous to administer an inositol combination to the subject in the form of a soft gel capsule, in which the soft gel capsule comprises MI and DCI in a respective MI:DCI weight ratio of about 40:1or is exactly 40:1, which is about the natural ratio of MI:DCI in the human body.

Especially the latter embodiments comprising administering about 2000 mg inositol(s), 2000 mg of inositol(s), about 3000 mg of inositol(s), or 3000 mg of inositol(s), are especially advantageous because such combined administration would be necessary only twice a day in order to reach a daily target amount of as much as about 6000 mg inositol(s), or about 4000 mg inositol(s). A combined administration entailing administration of about 2000 mg of Ml, in which the weight ratio of administered MI:DCI is e.g. about 40:1, implies a corresponding amount of administered DCI of about 50 mg. Combined administrations entailing administration of about 2000 mg of Ml, and about 50 mg of DCI are especially preferred as striking an ideal balance between therapeutic efficacy and acceptable impact on patient quality of life. Similarly, a combined administration entailing administration of about 550 mg of Ml, e.g. in a soft gel capsule, in which the weight ratio of administered MI:DCI is e.g. about 40:1, implies a corresponding amount of administered DCI, e.g. in the same soft gel capsule, of about 13.8 mg. Combined administrations entailing administration of about 550 mg of Ml, and about 13.8 mg of DCI are especially preferred as striking an ideal balance between therapeutic efficacy and acceptable impact on patient quality of life.

The following provides several examples illustrating various embodiments of the present invention, and the technical effects and advantages which it achieves. It should be understood that the following examples are presented for illustrative purposes only, and do not limit the claimed invention.

### Examples

### General

In the broadest sense, the present inventors have found that administration of inositol(s) can treat reactive muscle fibrosis, e.g. fibrosis caused by metabolic alteration, in particular cardiac fibrosis, e.g perivascular cardiac fibrosis, interstitial cardiac fibrosis or both perivascular and interstitial cardiac fibrosis. This represents a new and unexpected route to treat reactive muscle fibrosis, especially cardiac fibrosis.

### Example 1

### Effect of inositol administration (mixture of myoinositol (MI) and D-chiro inositol (DCI)) in pregnancies complicated by Metabolic Syndrome (MetS)

The present example describes a study in which a mice model of metabolic syndrome (MetS) was treated with a combination of inositols according to the present invention. The study aims to determine whether the combination of inositols is able to treat reactive muscle fibrosis, in particular cardiac fibrosis. A mouse model for MetS was used as the subject treatment group, because MetS is strongly associated with cardiac fibrosis and the mouse model allows the reproduction of a condition of high reactive fibrosis (interstitial and perivascular) and cardiovascular complications..

Hypertensive female mice, heterozygous for the endothelial nitric oxide synthase (eNOS) gene, fed high fat diet for 4 weeks develop a MS phenotype. Females were bred with wild type males. At gestational day (GD1), pregnant dams were randomly allocated to receive either the mixture of MI and DCI (equivalently referred to as "MI:DCI mixture") in water or water alone as a control.

On gestational day (GD) 1, metabolic-like syndrome mice were randomly allocated to receive either a mixture of MI and DCI dissolved in water (7.2 and 0.18 mg/mL, respectively, based on previous animal and human studies) or plain water as placebo control. On average, pregnant mice drink 5 mL/day, so daily consumption of MI and DCI was approximately 36 mg and 0.9 mg of MI and DCI per day per mouse, respectively (corresponding to a respective MI:DCI weight ratio of 40:1).

At GD18 pregnant dams were sacrificed. Maternal heart was collected under formalin (10%). Ventricular sections were obtained from control and treated pregnant MetS mice. Masson's trichrome was used to quantitatively evaluate connective tissue deposition in maternal heart according to previously reported methodology (Cohen A. H. "Masson's Trichrome Stain in the evaluation of renal biopsies", American Journal of Clinical Pathology, 64, 1976).

*Results:* Both immunohistochemical and quantitative analysis showed that cardiac interstitial and perivascular fibrosis were significantly reduced in MS dams after treatment with the mixture of MI and DCI.. The data obtained are graphically depicted in Figure 1.

| Table 1. **MetS Model:** Quantitative measurement of connective tissue in maternal heart (mean ± standard error of the mean) | | | |
|---|---|---|---|
| | **MI:DCI Mixture** | **Control** | **p value** |
| **Intestitial fibrosis (%)** | 2.57±0.8 | 21.1±2.9 | 0.001 |
| **Perivascular fibrosis (%)** | 4.67±1.3 | 19.9±2.4 | 0.001 |

As the above data indicate in Table 1, and as can clearly be seen in corresponding Figure 1, administration of a combination of inositols to the mice led to a dramatic decrease in both interstitial and perivascular cardiac fibrosis relative to untreated controls.

### Example 2

### Effectiveness of maternal administration of (mixture of myoinositol (MI) and D-chiro inositol (DCI)) in an obese pregnant mouse model

The present example describes a study in which a mice model of obesity was treated with a combination of inositols according to the present invention. The study aims to determine whether the combination of inositols is able to treat reactive muscle fibrosis, in particular cardiac fibrosis. A mouse model for obesity was used as the subject treatment group, because cardiac fibrosis is strongly associated with obesity and the mice model used reproduces a condition of high cardiac fibrosis and cardiovascular complications.

Female C57BL/6J WT mice at 3 weeks of age were placed on high fat diet for 4 weeks to obtain an obese mouse model phenotype. Then at 7-8 weeks of age, obese female mice were bred with WT males. At gestational day (GD) 1 pregnant dams were randomly allocated to receive either a mixture of MI and DCI (at a respective MI:DCI weight ratio of 40:1) dissolved in water or plain water, as placebo (control group).

At GD18, pregnant dams were sacrificed. Maternal heart was collected under formalin (10%). Ventricular sections were obtained from control and treated pregnant obese mice. Masson's trichrome was used to quantitatively evaluate connective deposition in maternal heart according to previously reported methodology (Cohen A. H. "Masson's Trichrome Stain in the evaluation of renal biopsies", American Journal of Clinical Pathology, 64, 1976).

*Results:* Maternal heart interstitial and perivascular fibrosis were significantly reduced in obese dams by treatment with a combination of MI and DCI. The data obtained are graphically depicted in Figure 2.

| Table 2. **Obesity Model:** Quantitative measurement of connective tissue in maternal heart (mean ± standard error of the mean) | | | |
|---|---|---|---|
| | **MI:DCI Mixture** | **Control** | **p value** |
| **Intestitial fibrosis (%)** | 2.35±1.5 | 11±1.1 | 0.001 |
| **Perivascular fibrosis (%)** | 8.52±2.8 | 19.0±3.7 | 0.001 |

As the above data indicate in Table 2, and as can clearly be seen in corresponding Figure 2, administration of a combination of inositols to the mice led to a dramatic decrease in both interstitial and perivascular cardiac fibrosis relative to untreated controls.

## Claims

1. Inositol for use in a method of treating or preventing reactive muscle fibrosis in a subject.

2. The inositol for use as in claim 1, wherein said reactive muscle fibrosis is cardiac fibrosis.

3. The inositol for use as in claim 2, wherein said cardiac fibrosis is interstitial cardiac fibrosis, perivascular cardiac fibrosis or both interstitial and perivascular cardiac fibrosis.

4. The inositol for use as in any one of claims 1-3, wherein said inositol is myoinositol.

5. The inositol for use as in any one of claims 1-3, wherein said inositol is a combination of myoinositol and at least one isomer of inositol other than myoinositol.

6. The inositol for use as in claim 5, wherein said method comprises administering said myoinositol and said at least one isomer of inositol other than myoinositol to said subject simultaneously or non-simultaneously.

7. The inositol for use as in claim 6, wherein said method comprises administering said myoinositol and said at least one isomer of inositol other than myoinositol to said subject simultaneously, and wherein said myoinositol and said at least one isomer of inositol other than myoinositol are present in a composition comprising said myoinositol and said at least one isomer of inositol other than myoinositol.

8. The inositol for use as in claim 7, wherein said composition is a pharmaceutical composition additionally comprising at least one pharmaceutically acceptable ingredient.

9. The inositol for use as any one of claims 5-8, wherein said at least one isomer of inositol other than myoinositol comprises D-chiroinositol.

10. The inositol for use as in claim 9, wherein said method comprises administering myoinositol and D-chiroinositol to said subject in a respective weight ratio of about 10:1 to about 100:1, about 20:1 to about 80:1, about 30:1 to about 60:1, about 35:1 to about 50:1, about 40:1 or 40:1.

11. The inositol for use as any one of claims 1-10, wherein said method comprises administering to the subject
• about 500-4000 mg myoinositol;
• about 1000-2000 mg myoinositol;
• about 2000 mg myoinositol;
• about 600 mg myoinositol;
• about 550 mg myoinositol;
• about 550 mg myoinositol and about 13.8 mg D-chiroinositol; or
• about 2000 mg myoinositol and about 50 mg D-chiroinositol.

12. The inositol for use as in either of claims 9 or 10, wherein said method comprises administering to the subject
• about 5-100 mg D-chiroinositol;
• about 10-50 mg D-chiroinositol;
• about 50 mg D-chiroinositol; or
• about 13.8 mg D-chiroinositol.

13. The inositol for use as in any one of the preceding claims, wherein said method comprises administering to the subject a total of about 2050 mg total inositol in the form of a powder.

14. The inositol for use as in claim 13, wherein said about 2050 mg total inositol is constituted by about 2000 mg myoinositol and about 50 mg D-chiroinositol.

15. The inositol for use as in any one of the preceding claims, wherein said method comprises administering to the subject a total of about 563.8 mg total inositol in the form of a soft gel capsule, preferably said about 563.8 mg total inositol is constituted by about 550 mg myoinositol and about 13.8 mg D-chiroinositol.

## Patentansprüche

1. Inositol zur Verwendung in einem Verfahren zur Behandlung oder Prävention von reaktiver Muskelfibrose bei einem Subjekt.

2. Inositol zur Verwendung nach Anspruch 1, wobei die reaktive Muskelfibrose Herzfibrose ist.

3. Inositol zur Verwendung nach Anspruch 2, wobei die Herzfibrose interstitielle Herzfibrose, perivaskuläre Herzfibrose oder sowohl interstitielle als auch perivaskuläre Herzfibrose ist.

4. Inositol zur Verwendung nach einem der Ansprüche 1 bis 3, wobei das Inositol Myoinositol ist.

5. Inositol zur Verwendung nach einem der Ansprüche 1 bis 3, wobei das Inositol eine Kombination von Myoinositol und mindestens einem Isomer von Inositol ist, das von Myoinositol verschieden ist.

6. Inositol zur Verwendung nach Anspruch 5, wobei das Verfahren Verabreichen des Myoinositols und des mindestens einen Isomers von Inositol, das von Myoinositol verschieden ist, an das Subjekt in simultaner oder nicht-simultaner Weise umfasst.

7. Inositol zur Verwendung nach Anspruch 6, wobei das Verfahren Verabreichen des Myoinositols und des mindestens einen Isomers von Inositol, das von Myoinositol verschieden ist, an das Subjekt in simultaner Weise umfasst, und wobei das Myoinositol und das mindestens eine Isomer von Inositol, das von Myoinositol verschieden ist, in einer Zusammensetzung vorhanden sind, die das Myoinositol und das mindestens eine Isomer von Inositol, das von Myoinositol verschieden ist, umfasst.

8. Inositol zur Verwendung nach Anspruch 7, wobei die Zusammensetzung eine pharmazeutische Zusammensetzung ist, die zusätzlich mindestens einen pharmazeutisch annehmbaren Bestandteil umfasst.

9. Inositol zur Verwendung nach einem der Ansprüche 5 bis 8, wobei das mindestens eine Isomer von Inositol, das von Myoinositol verschieden ist, D-Chiroinositol umfasst.

10. Inositol zur Verwendung nach Anspruch 9, wobei das Verfahren Verabreichen von Myoinositol und D-Chiroinositol an das Subjekt in einem jeweiligen Gewichtsverhältnis von etwa 10:1 bis etwa 100:1, etwa 20:1 bis etwa 80:1, etwa 30:1 bis etwa 60:1, etwa 35:1 bis etwa 50:1, etwa 40:1 oder 40:1 umfasst.

11. Inositol zur Verwendung nach einem der Ansprüche 1 bis 10, wobei das Verfahren Verabreichen von folgendem an das Subjekt umfasst:
• etwa 500 bis 4000 mg Myoinositol;
• etwa 1000 bis 2000 mg Myoinositol;
• etwa 2000 mg Myoinositol;
• etwa 600 mg Myoinositol;
• etwa 550 mg Myoinositol;
• etwa 550 mg Myoinositol und etwa 13,8 mg D-Chiroinositol; oder
• etwa 2000 mg Myoinositol und etwa 50 mg D-Chiroinositol.

12. Inositol zur Verwendung nach einem der Ansprüche 9 oder 10, wobei das Verfahren Verabreichen von folgendem an das Subjekt umfasst:
• etwa 5 bis 100 mg D-Chiroinositol;
• etwa 10 bis 50 mg D-Chiroinositol;
• etwa 50 mg D-Chiroinositol; oder
• etwa 13,8 mg D-Chiroinositol.

13. Inositol zur Verwendung nach einem der vorhergehenden Ansprüche, wobei das Verfahren Verabreichen von insgesamt etwa 2050 mg Gesamtinositol in Form eines Pulvers an das Subjekt umfasst.

14. Inositol zur Verwendung nach Anspruch 13, wobei die etwa 2050 mg Gesamtinositol durch etwa 2000 mg Myoinositol und etwa 50 mg D-Chiroinositol gestellt werden.

15. Inositol zur Verwendung nach einem der vorhergehenden Ansprüche, wobei das Verfahren Verabreichen von insgesamt etwa 563,8 mg Gesamtinositol in Form einer Weichgelkapsel an das Subjekt umfasst, wobei vorzugsweise die etwa 563,8 mg Gesamtinositol durch etwa 550 mg Myoinositol und etwa 13,8 mg D-Chiroinositol gestellt werden.

## Revendications

1. Inositol destiné à être utilisé dans le traitement de la fibrose musculaire réactive chez un sujet.

2. Inositol destiné à être utilisé selon la revendication 1, dans lequel ladite fibrose musculaire réactive est la fibrose cardiaque.

3. Inositol destiné à être utilisé selon la revendication 2, dans lequel ladite fibrose cardiaque est la fibrose cardiaque interstitielle, la fibrose cardiaque périvasculaire ou les deux fibroses cardiaques interstitielle et périvasculaire.

4. Inositol destiné à être utilisé selon l'une quelconque des revendications 1 à 3, dans lequel ledit inositol est le myoinositol.

5. Inositol destiné à être utilisé selon l'une quelconque des revendications 1 à 3, dans lequel ledit inositol est une combinaison de myoinositol et d'au moins un isomère de l'inositol différent du myoinositol.

6. Inositol destiné à être utilisé selon la revendication 5, dans lequel ledit procédé comprend l'administration dudit myoinositol et dudit au moins un isomère de l'inositol différent du myoinositol audit sujet simultanément ou non simultanément.

7. Inositol destiné à être utilisé selon la revendication 6, dans lequel ledit procédé comprend l'administration dudit myoinositol et dudit au moins un isomère de l'inositol différent du myoinositol audit sujet simultanément et ledit myoinositol et ledit au moins un isomère d'inositol différent du myoinositol étant présents dans une composition comprenant ledit myoinositol et ledit au moins un isomère d'inositol différent du myoinositol.

8. Inositol destiné à être utilisé selon la revendication 7, dans lequel ladite composition est une composition pharmaceutique comprenant de plus au moins un ingrédient pharmaceutiquement acceptable.

9. Inositol destiné à être utilisé selon l'une quelconque des revendications 5 à 8, dans lequel ledit au moins un isomère d'inositol différent du myoinositol comprend du D-chiroinositol.

10. Inositol destiné à être utilisé selon la revendication 9, dans lequel ledit procédé comprend l'administration de myoinositol et de D-chiroinositol audit sujet dans un rapport en poids respectif d'environ 10 : 1 à environ 100 : 1, d'environ 20 : 1 à environ 80 : 1, d'environ 30 : 1 à environ 60 : 1, d'environ 35 : 1 à environ 50 : 1, d'environ 40 : 1 à environ 40 : 1.

11. Inositol destiné à être utilisé selon l'une quelconque des revendications 1 à 10, dans lequel ledit procédé comprend l'administration au sujet
• d'environ 500 à 4 000 mg de myoinositol ;
• d'environ 1 000 à 2 000 mg de myoinositol ;
• d'environ 2 000 mg de myoinositol ;
• d'environ 600 mg de myoinositol ;
• d'environ 550 mg de myoinositol ;
• d'environ 550 mg de myoinositol et d'environ 13,8 mg de D-chiroinositol ; ou
• d'environ 2 000 mg de myoinositol et d'environ 50 mg de D-chiroinositol.

12. Inositol destiné à être utilisé selon l'une des revendications 9 ou 10, dans lequel ledit procédé comprend l'administration au sujet
• d'environ 5 à 100 mg de D-chiroinositol ;
• d'environ 10 à 50 mg de D-chiroinositol ;
• d'environ 50 mg de D-chiroinositol ; ou
• d'environ 13,8 mg de D-chiroinositol.

13. Inositol destiné à être utilisé selon l'une quelconque des revendications précédentes, dans lequel ledit procédé comprend l'administration au sujet d'un total d'environ 2 050 mg d'inositol total sous forme de poudre.

14. Inositol destiné à être utilisé selon la revendication 13, dans lequel lesdits environ 2 050 mg d'inositol total sont constitués d'environ 2 000 mg de myoinositol et d'environ 50 mg de D-chiroinositol.

15. Inositol destiné à être utilisé selon l'une quelconque des revendications précédentes, dans lequel ledit procédé comprend l'administration au sujet d'un total d'environ 563,8 mg d'inositol total sous la forme d'une capsule de gel molle, de préférence lesdits 563,8 mg d'inositol total sont constitués d'environ 550 mg de myoinositol et d'environ 13,8 mg de D-chiroinositol.
